# EUROPÄISCHE PATENTSCHRIFT

(11) **EP 0 884 028 B1**
(45) Veröffentlichungstag und Bekanntmachung des Hinweises auf die Patenterteilung: **27.02.2002**
(21) Anmeldenummer: 98108486.6
(22) Anmeldetag: 09.05.1998
(51) Int. Cl.: A61F 2/06

(54) **Kombination aus einem radial aufweitbaren Stent zur Implantierung in ein Körpergefäss im Bereich einer Gefässverzweigung und einem Ballonkatheter**
Combination of a radially dilatable stent for placement around a bifurcating corporeal vessel with a balloon catheter
Combinaison d'un stent radialement dilatable pour le placement dans la zone bifurquée d'un vaisseau corporel avec un cathéter-ballon

(30) Priorität: 17.05.1997 DE 29708803 U
(43) Veröffentlichungstag der Anmeldung: 16.12.1998
(73) Patentinhaber: Jomed Implantate GmbH, 72414 Rangendingen (DE)
(72) Erfinder: von Oepen, Randolf, Dr.-Ing., 72145 Hirrlingen (DE)
(74) Vertreter: Schmitz, Hans-Werner, Dipl.-Ing.

(56) Entgegenhaltungen:
- DE-U- 29 701 758
- US-A- 4 994 071
- US-A- 5 609 627

## Beschreibung

Die Erfindung betrifft eine Kombination aus einem radial aufweitbaren Stent zur Implantierung in ein Körpergefäß im Bereich einer Gefäßverzweigung in Form eines hohlzylindrischen Elements und einem Ballonkatheter wie im Oberbegriff des Anspruchs 1. An Verengungsstellen in Körpergefäßen oder Körperhöhlungen werden heutzutage zur Aufweitung der Verengung und Stabilisierung der Gefäßwand radial aufweitbare Stents eingesetzt. Solche Verengungen von Körpergefäßen können dabei auch im Bereich von Gefäßverzweigungen auftreten. Hier ist der Einsatz herkömmlicher Stents nicht möglich, da deren Wandung den freien Blutdurchfluß in das abzweigende Gefäß behindern würde. In der DE 297 01 758.6, der dem Oberbegriff des Anspruchs 1 entspricht, wurde daher ein spezieller Stent vorgeschlagen, der einen Abschnitt mit vergrößerten radialen öffnungen aufweist, so daß dieser Abschnitt über die Abzweigungsstelle des Seitenastgefäßes gelegt werden kann und den Blutdurchfluß nicht mehr oder nur noch geringfügig behindert. Bei einer entsprechend ungünstigen Stenosenbildung des Hauptgefäßes direkt im Abzweigungsbereich kann dieser Stent aufgrund der sich über einen ganzen Abschnitt erstreckenden großen radialen öffnungen den erkrankten Gefäßabschnitt jedoch nicht genügend abdecken.

Der vorliegenden Erfindung liegt daher die Aufgabe zugrunde, einen Stent zu schaffen, der im Bereich von Gefäßverzweigungen einsetzbar ist und dabei die obengenannten Nachteile vermeidet.

Die Aufgabe wird mit einer Kombination aus einem Stent und einem Ballonkatheter gemäß dem Hauptanspruch gelöst. Aufgrund der lediglich einen vergrößerten öffnung in der Stentwandung, die direkt über die Abzweigungsstelle gelegt werden kann, wird sichergestellt, daß die gesamte Gefäßwand durch den Stent sicher abgestützt wird. Der vergrößerten öffnung kommt hierbei die Aufgabe zu, den ungehinderten Blutfluß in das Seitenastgefäß zu garantieren. Zur genauen Positionierung dieses Stents sind jedoch unbedingt entsprechende Hilfsmittel nötig. Allein aufgrund der Verwendung von Röntgenkontrastmitteln ist die exakte Positionierung der einen vergrößerten öffnung über der Abzweigung nicht möglich. Deswegen ist der erfindungsgemäße Stent auf einem Ballonkatheter vormontiert, der einen Hohlraum für einen Führungsdraht aufweist, der durch die vergrößerte öffnung austritt. Mit Hilfe dieses Führungsdrahtes ist es möglich, die große öffnung des Stents genau über dem Abgang des Seitengefäßes zu positionieren. Hierzu wird der Katheter durch Drehen und Schieben unter Zugabe von Röntgenkontrastmittel und visueller Beobachtung auf dem Röntgenschirm so lange manipuliert, bis es möglich ist, den zweiten Führungsdraht in das Seitenastgefäß einzuführen. Zur Einführung des Ballonkatheters in das Hauptgefäß kann der Ballonkatheter in an sich bekannter Weise einen entlang seiner Längsachse verlaufenden Hohlraum für einen an der Spitze des Ballonkatheters austretenden ersten Führungsdraht aufweisen. Entlang dieses Führungsdrahtes kann er problemlos bis zur Gefäßabzweigung vorgeschoben werden, bevor mit Hilfe des zweiten Führungsdrahtes die exakte Positionierung der vergrößerten Öffnung vorgenommen wird. Nachdem sichergestellt ist, daß der Katheter exakt positioniert ist, wird der Ballon aufgeblasen, und der Stent legt sich an die Gefäßwandung an. Anschließend kann der Katheter entlang der beiden Führungsdrähte wieder aus dem Gefäß herausgezogen werden. Der Stent verbleibt mit den beiden Drähten im Gefäß. Über den Führungsdraht des Seitenastgefäßes kann ein weiterer Ballonkatheter in die Verzweigung eingeführt werden, um die vergrößerte öffnung erforderlichenfalls nachzudehnen. Somit kann gewährleistet werden, daß kein Wandungsteil des Stents den Blutfluß in das Seitenastgefäß behindert. Für die Ausbildung des Ballonkatheters gibt es unterschiedliche Möglichkeiten. So kann der Hohlraum für den aus der vergrößerten öffnung herausgeführten Führungsdraht durch Befestigen eines Röhrchens auf der Ballonoberfläche gebildet werden. Dieser Hohlraum kann jedoch auch durch den Zwischenraum eines doppelwandigen Ballons gebildet sein. Auch das überziehen eines dehnbaren Schlauchstückes bis über den Ballon ist möglich, um einen Hohlraum zwischen diesem übergezogenen Schlauchstück und dem Ballon für den Führungsdraht zu schaffen. Bei einer alternativen Ausgestaltung kann der Ballonkatheter drei koaxial angeordnete Schläuche aufweisen, wobei die beiden innenliegenden Schläuche Hohlräume zur Aufnahme der beiden Führungsdrähte bilden.

Auch der Stent selbst kann auf unterschiedliche Art und Weise ausgebildet sein. Er kann eine multizellulare Wandung aufweisen und aus einem Rohr gefertigt sein. Aber auch das Fertigen des Stents aus Draht ist möglich. Dabei kann er aus Draht gebogen, geflochten, gestrickt oder gewirkt sein. Zweckmäßigerweise kann die vergrößerte öffnung in der Mitte des Stents angeordnet sein, jedoch ist auch eine außermittige Anordnung der vergrößerten öffnung für bestimmte Anwendungsfälle realisierbar.

Im folgenden wird ein bevorzugtes Ausführungsbeispiel eines erfindungsgemäßen Stents anhand der Zeichnung näher beschrieben.

Es zeigen:
- Fig. 1: drei schematische Darstellungen von Gefäßverzweigungen mit Stenosen;
- Fig. 2: eine Darstellung der Oberflächenstruktur eines erfindungsgemäßen Stents;
- Fig. 3: eine Seitenansicht eines auf einen Ballonkatheter vormontierten Stents.

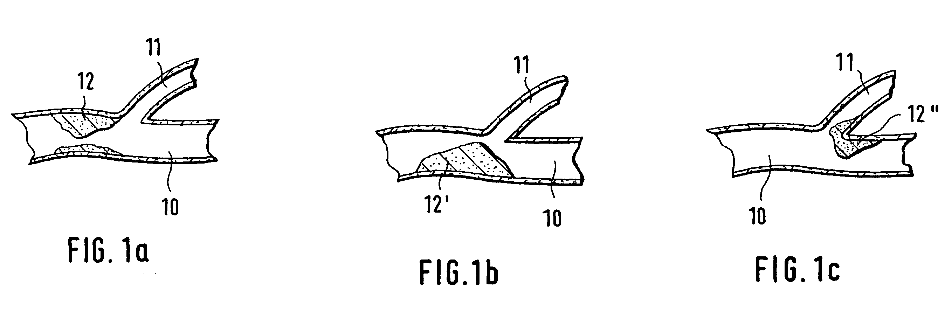

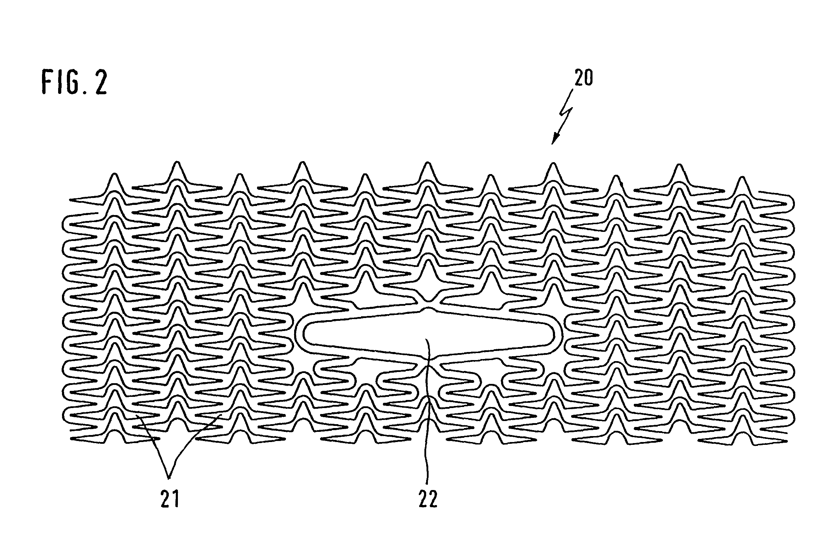

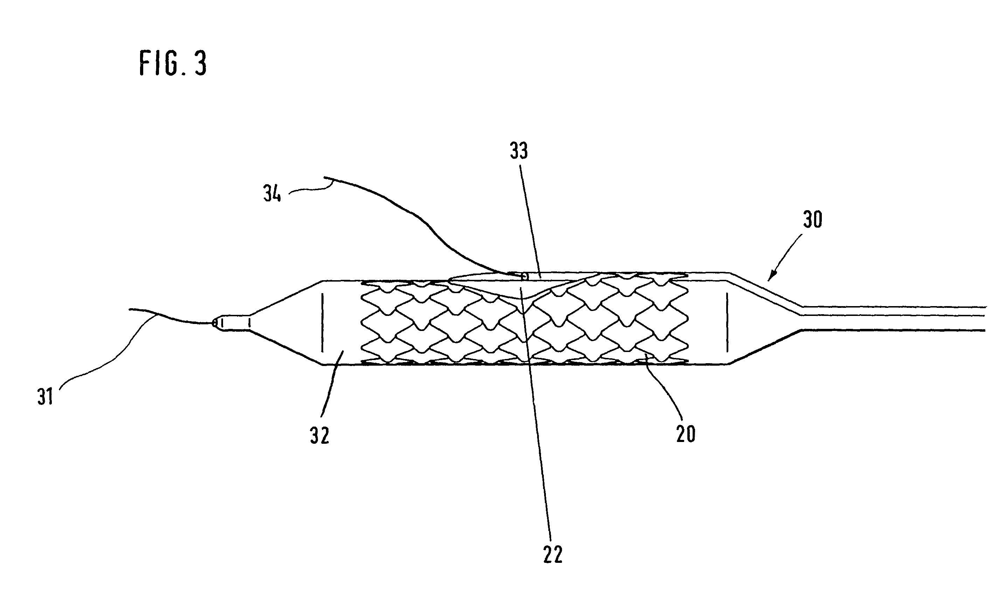

Fig. 1 zeigt drei Beispiele typischer Stenosen, wie sie an Gefäßverzweigungen auftreten können. In Fig. 1a befindet sich die Stenose 12 im Hauptgefäß 10 vor der Abzweigung eines Seitenastgefäßes 11. In Fig. 1b liegt eine sehr große Stenose 12' direkt gegenüber der Abzweigung des Seitenastgefäßes 11 und in Fig. 1c im Übergang zwischen Hauptgefäß 10 und Seitenastgefäß 11. Insbesondere die Stenose nach Fig. 1b ist mit einem Bifurkations-Stent mit einem Abschnitt mit vergrößerten radialen öffnungen nur unzureichend abzudecken. Gerade für solche Fälle eignet sich ein erfindungsgemäßer Stent, wie er in den Fig. 2 und 3 dargestellt ist. Fig. 2 zeigt die Oberflächenstruktur eines Stents 20, der eine Vielzahl von im gedehnten Zustand rautenförmigen radialen öffnungen 21 aufweist. Im mittleren Bereich des Stents 20 ist eine einzige, sehr große rautenförmige öffnung 22 eingeformt, die in einem Körpergefäß genau über die Abzweigung eines Seitenastgefäßes 11 (Fig. 1) gelegt werden kann. Zur Ermöglichung der lagegenauen Positionierung des Stents 20 ist dieser, wie in Fig. 3 gezeigt ist, auf einem Ballonkatheter 30 vormontiert. Der in Fig. 3 gezeigte Ballonkatheter 30 weist in seinem Inneren einen nicht näher dargestellten Hohlraum zum Durchführen eines ersten Führungsdrahtes 31 auf. Im Bereich eines Ballons 32 des Katheters 30 ist ein Stent 20 aufgezogen. Bis in den Bereich des Ballons 32 erstreckt sich außerdem ein weiterer Hohlraum 33 zum Hindurchführen eines zweiten Führungsdrahtes 34, der im Bereich der vergrößerten öffnung 22 aus dem Hohlraum 33 und aus dem Stent 20 austritt. Dieser Führungsdraht 34 wird in ein Seitenastgefäß 11 eingeführt und dient somit als Justierhilfe für die Positionierung des Stents 20 in einem Körpergefäß 10. Er kann außerdem, nach Herausziehen des Ballonkatheters 30 aus dem Gefäß 10, als Führungsdraht für einen weiteren Ballonkatheter zur Weitung der vergrößerten öffnung 22 eingesetzt werden. Die dargestellten Ausführungsformen des Ballonkatheters 30 sowie des Stents 20 sind lediglich beispielhaft. Der notwendige Hohlraum 33 zur Durchführung des zweiten Führungsdrahtes 34 kann beispielsweise auch durch einen doppelwandigen Ballon 32 oder zwei koaxiale Schlauchstücke gebildet werden. Der dargestellte Stent 20 ist aus einem Röhrchen geschnitten; er kann jedoch auch aus Draht gebogen, gestrickt, geflochten oder gewirkt werden.

## Patentansprüche

1. Kombination aus einem radial aufweitbaren Stent zur Implantierung in ein Körpergefäß im Bereich einer Gefäßverzweigung in Form eines hohlzylindrischen Elements und einem Ballonkatheter (30), der einen Hohlraum (33) zum Hindurchführen eines Führungsdrahtes (34) aufweist, **dadurch gekennzeichnet, daß** der Stent in seinem nicht expandierten Zustand lediglich eine vergrößerte radiale Öffnung (22) aufweist und zur Implantierung in das Gefäß (10) derart auf dem Ballonkatheter (30) vormontiert ist, daß der Führungsdraht (34) im Zentrum der vergrößerten Öffnung (22) aus dem Hohlraum (33) und dem Stent (20) austritt.

2. Kombination nach Anspruch 1, **dadurch gekennzeichnet, daß** der Ballonkatheter (30) einen entlang seiner Längsachse verlaufenden Hohlraum für einen an der Spitze des Ballonkatheters (30) austretenden Führungsdraht (31) aufweist.

3. Kombination nach Anspruch 1 und 2, **dadurch gekennzeichnet, daß** der Ballonkatheter (30) nach der Dilatation des Stents (20) entlang der beiden Führungsdrähte (31, 34) aus dem Gefäß (10) herausziehbar ist.

4. Kombination nach einem der Ansprüche 1 bis 3, **dadurch gekennzeichnet, daß** der Hohlraum (33) für den aus der vergrößerten öffnung (22) herausgeführten Führungsdraht (34) durch Befestigen eines Röhrchens auf der Ballonoberfläche gebildet wird.

5. Kombination nach einem der Ansprüche 1 bis 3, **dadurch gekennzeichnet, daß** der Hohlraum (33) für den aus der vergrößerten öffnung (22) herausgeführten Führungsdraht (34) durch den Zwischenraum eines doppelwandigen Ballons (32) gebildet wird.

6. Kombination nach einem der Ansprüche 1 bis 3, **dadurch gekennzeichnet, daß** der Hohlraum (33) für den aus der vergrößerten öffnung (22) herausgeführten Führungsdraht (34) durch den Zwischenraum eines über den Ballon (32) gezogenen, dehnbaren Schlauchstücks und dem Ballon (32) gebildet wird.

7. Kombination nach einem der Ansprüche 1 bis 3, **dadurch gekennzeichnet, daß** der Ballonkatheter (30) drei koaxial angeordnete Schläuche aufweist, wobei die beiden innenliegenden Schläuche Hohlräume zur Aufnahme der beiden Führungsdrähte (31, 34) bilden.

8. Kombination nach einem der Ansprüche 1 bis 7, **dadurch gekennzeichnet, daß** er aus einem Rohr gefertigt ist und eine multizellulare Wandung aufweist.

9. Kombination nach einem der Ansprüche 1 bis 7, **dadurch gekennzeichnet, daß** er aus Draht gebogen ist.

10. Kombination nach einem der Ansprüche 1 bis 7, **dadurch gekennzeichnet, daß** er aus Draht geflochten, gestrickt oder gewirkt ist.

11. Kombination nach einem der Ansprüche 1 bis 10, **dadurch gekennzeichnet, daß** die vergrößerte öffnung (22) in der Stent-Mitte angeordnet ist.

12. Kombination nach einem der Ansprüche 1 bis 10, **dadurch gekennzeichnet, daß** die vergrößerte öffnung (22) außermittig am Stent (20) angeordnet ist.

## Claims

1. A combination consisting of a radially dilatable stent for implantation into a corporeal vessel in the region of a vascular bifurcation in the form of a hollow cylindrical element and a balloon catheter (30), which comprises a cavity (33) for passing through a guide wire (34),
**characterised in that** in its non-expanded condition the stent comprises just one enlarged radial opening (22) and for implantation into the vessel (10) is premounted on the balloon catheter (30) in such a manner that the guide wire (34) exits from the cavity (33) and the stent (20) in the centre of the enlarged opening (22).

2. A combination according to Claim 1,
**characterised in that** the balloon catheter (30) comprises a cavity extending along its longitudinal axis for a guide wire (31) exiting at the tip of the balloon catheter (30).

3. A combination according to Claim 1 and 2,
**characterised in that** balloon catheter (30) can be pulled out of the vessel (10) along the two guide wires (31, 34) after the dilation of the stent (20).

4. A combination according to one of Claims 1 to 3,
**characterised in that** the cavity (33) for the guide wire (34) passed out of the enlarged opening (22) is formed by attaching a small tube to the surface of the balloon.

5. A combination according to one of Claims 1 to 3,
**characterised in that** the cavity (33) for the guide wire (34) passed out of the enlarged opening (22) is formed by the space formed by a double-walled balloon (32).

6. A combination according to one of Claims 1 to 3,
**characterised in that** the cavity (33) for the guide wire (34) passed out of the enlarged opening (22) is formed by the space between an elastic tubular piece pulled over the balloon (32) and the balloon (32) itself.

7. A combination according to one of Claims 1 to 3,
**characterised in that** the balloon catheter (30) comprises three coaxially disposed hoses, the two inner hoses of which form cavities for receiving the two guide wires (31, 34).

8. A combination according to one of Claims 1 to 7,
**characterised in that** it is produced from a tube and has a multicellular wall.

9. A combination according to one of Claims 1 to 7,
**characterised in that** it is bent from wire.

10. A combination according to one of Claims 1 to 7,
**characterised in that** it is braided, knitted or machine-knitted from wire.

11. A combination according to one of Claims 1 to 10,
**characterised in that** the enlarged opening (22) is disposed in the centre of the stent.

12. A combination according to one of Claims 1 to 10,
**characterised in that** the enlarged opening (22) is disposed eccentrically on the stent (20).

## Revendications

1. Combinaison d'un extenseur radialement dilatable pour l'implantation dans la zone bifurquée d'un vaisseau sous la forme d'un élément cylindrique creux et d'un cathéter-ballon (30) qui présente un espace creux (33) pour l'introduction d'un fil de guidage (34), **caractérisée en ce que** l'extenseur dans son état non dilaté présente simplement une ouverture radiale agrandie (22) et est pré-monté sur le cathéter-ballon (30) pour l'implantation dans le vaisseau (10) de telle sorte que le fil de guidage (34) sort de l'espace creux (33) et de l'extenseur (20) au centre de l'ouverture agrandie (22).

2. Combinaison selon la revendication 1, **caractérisée en ce que** le cathéter-ballon (30) présente un espace creux le long de son axe longitudinal pour un fil de guidage (31) sortant de l'extrémité du cathéter-ballon (30).

3. Combinaison selon les revendications 1 et 2, **caractérisée en ce que** le cathéter-ballon (30) est extractible du vaisseau (10) après dilatation de l'extenseur (20) le long des deux fils de guidage (31, 34).

4. Combinaison selon l'une des revendications 1 à 3, **caractérisée en ce que** l'espace creux (33) pour le fil de guidage (34) sortant de la grande ouverture (22) est formé par fixation d'un tube à la surface du ballon.

5. Combinaison selon l'une des revendications 1 à 3, **caractérisée en ce que** l'espace creux (33) pour le fil de guidage (34) sortant de l'ouverture agrandie (22) est formé par l'intervalle d'un ballon à double paroi (32).

6. Combinaison selon l'une des revendications 1 à 3, **caractérisée en ce que** l'espace creux (33) pour le fil de guidage (34) sortant de l'ouverture agrandie (22) est formé par l'intervalle entre le morceau de tuyau extensible couvrant le ballon (32) et le ballon (32).

7. Combinaison selon l'une des revendications 1 à 3, **caractérisée en ce que** le cathéter-ballon (30) présente trois tuyaux disposés coaxialement, dont les deux tuyaux situés vers l'intérieur forment l'espace creux pour le passage des deux fils de guidage (31, 34).

8. Combinaison selon l'une des revendications 1 à 7, **caractérisée en ce que** l'extenseur est fabriqué à partir d'un tube et présente une paroi multicellulaire.

9. Combinaison selon l'une des revendications 1 à 7, **caractérisée en ce que** ses fils sont coudés.

10. Combinaison selon l'une des revendications 1 à 7, **caractérisée en ce que** ses fils sont tressés, maillés ou tissés.

11. Combinaison selon l'une des revendications 1 à 10, **caractérisée en ce que** l'ouverture agrandie (22) est disposée au centre de l'extenseur.

12. Combinaison selon l'une des revendications 1 à 7, **caractérisée en ce que** l'ouverture agrandie (22) est disposée de façon excentrée sur l'extenseur (20).
